# EUROPEAN PATENT APPLICATION

(11) **EP 1 688 137 A1**
(43) Date of publication of application: **09.08.2006**
(21) Application number: 05001843.1
(22) Date of filing: 28.01.2005
(51) Int. Cl.: A61K 31/165, A61P 25/18

(54) **SPM 927 for add-on therapy of schizophrenia**

(71) Applicant: SCHWARZ PHARMA AG, 40789 Monheim (DE)
(72) Inventor: Stöhr, Dr. rer. nat Thomas, 40789 Monheim (DE)
(74) Representative: Weiss, Wolfgang

(57) **Abstract**

The present invention is directed to the use of a class of peptide compounds for treating schizophrenia in an add-on therapy.

## Description

The present invention is directed to the use of a class of peptide compounds for treating schizophrenia in an add-on therapy.

Certain peptides are known to exhibit central nervous system (CNS) activity and are useful in the treatment of epilepsy and other CNS disorders. These peptides which are described in the U.S. Patent No. 5,378,729 have the Formula (la): 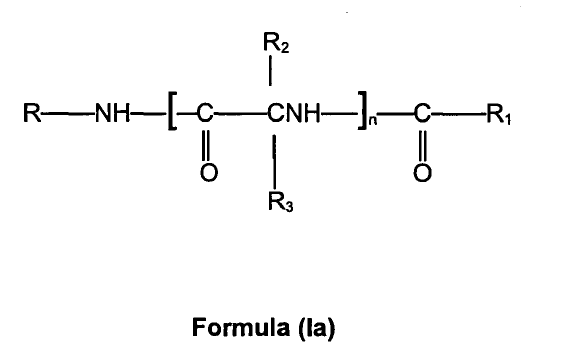 wherein
R is hydrogen, lower alkyl, lower alkenyl, lower alkynyl, aryl, aryl lower alkyl, heterocyclic, heterocyclic lower alkyl, lower alkyl heterocyclic, lower cycloalkyl, lower cycloalkyl lower alkyl, and R is unsubstituted or is substituted with at least one electron withdrawing group or electron donating group;
R₁ is hydrogen or lower alkyl, lower alkenyl, lower alkynyl, aryl lower alkyl, aryl, heterocyclic lower alkyl, heterocyclic, lower cycloalkyl, lower cycloalkyl lower alkyl, each unsubstituted or substituted with an electron donating group or an electron withdrawing group; and
R₂ and R₃ are independently hydrogen, lower alkyl, lower alkenyl, lower alkynyl, aryl lower alkyl, aryl, heterocyclic, heterocyclic lower alkyl, lower alkyl heterocyclic, lower cycloalkyl, lower cycloalkyl lower alkyl, or Z-Y wherein R₂ and R₃ may be unsubstituted or substituted with at least one electron withdrawing group or electron donating group;
Z is O, S, S(O)ₐ, NR₄, PR₄ or a chemical bond;
Y is hydrogen, lower alkyl, aryl, aryl lower alkyl, lower alkenyl, lower alkynyl, halo, heterocyclic, heterocyclic lower alkyl, and Y may be unsubstituted or substituted with an electron donating group or an electron withdrawing group, provided that when Y is halo, Z is a chemical bond, or
ZY taken together is NR₄NR₅R₇, NR₄OR₅, ONR₄R₇, OPR₄R₅, PR₄OR₅, SNR₄R₇, NR₄SR₇, SPR₄R₅ or PR₄SR₇, NR₄PR₅R₆ or PR₄NR₅R₇, 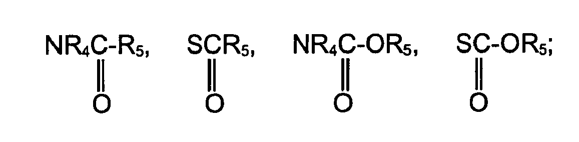 R₄, R₅ and R₆ are independently hydrogen, lower alkyl, aryl, aryl lower alkyl, lower alkenyl, or lower alkynyl, wherein R₄, R₅ and R₆ may be unsubstituted or substituted with an electron withdrawing group or an electron donating group; and
R₇ is Rₑ or COOR₈ or COR₈;
R₈ is hydrogen or lower alkyl, or aryl lower alkyl, and the aryl or alkyl group may be unsubstituted or substituted with an electron withdrawing group or an electron donating group; and
n is 1-4; and
a is 1-3.

U.S. Patent No. 5,773,475 also discloses additional compounds useful for treating CNS disorders. These compounds are N-benzyl-2-amino-3-methoxy-propionamide having the Formula (IIa): 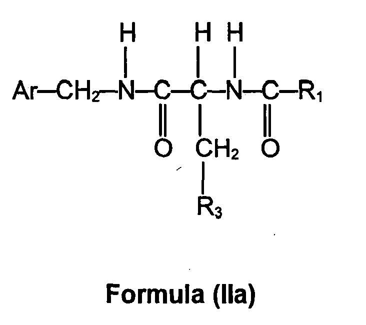 wherein
Ar is aryl which is unsubstituted or substituted with halo; R₃ is lower alkoxy; and R₁ is methyl.

The patents US 5.378.729 and US 5.773.475 are hereby incorporated by reference. However, neither of these patents describes the use of these compounds for treating treating schizophrenia in an add-on therapy.

WO 02/074297 relates to the use of a compound according to Formula (IIa) wherein Ar is phenyl which may be substituted by at least one halo, R₃ is lower alkoxy containing 1-3 carbon atoms and R₁ is methyl for the preparation of pharmaceutical compositions useful for the treatment of allodynia related to peripheral neuropathic pain.

WO 02/074784 relates to the use of a compound having Formula (la) or/and Formula (IIa) showing antinociceptive properties for treating different types and symptoms of acute and chronic pain, especially non neuropathic inflammatory pain, e.g. rheumatoid arthritic pain or/and secondary inflammatory osteo-arthritic pain.

Schizophrenia is a chronic, severe, and disabling brain disease found all over the world. Approximately 1 percent of the population develops schizophrenia during their lifetime. The severity of the symptoms and long-lasting, chronic pattern of schizophrenia often cause a high degree of disability. People with schizophrenia have a higher rate of suicide than the general population. Approximately 10 percent of people with schizophrenia (especially younger adult males) commit suicide. The first signs of schizophrenia often appear as confusing, or even shocking, changes in behavior. This sudden onset of severe psychotic symptoms is referred to as an "acute" phase of schizophrenia. "Psychosis," a common condition in schizophrenia, is a state of mental impairment marked by following symptoms:

### • Distorted Perceptions of Reality

People with schizophrenia may have perceptions of reality that are strikingly different from the reality seen and shared by others around them.

### • Hallucinations and Illusions

Hallucinations are perceptions that occur without connection to an appropriate source. Although hallucinations can occur in any sensory form - auditory (sound), visual (sight), tactile (touch), gustatory (taste), and olfactory (smell) - hearing voices that other people do not hear is the most common type of hallucination in schizophrenia.

### • Delusions

Delusions are false personal beliefs that are not subject to reason or contradictory evidence and are not explained by a person's usual cultural concepts.

### • Disordered Thinking

Schizophrenia often affects a person's ability to "think straight." Thoughts may come and go rapidly; the person may not be able to concentrate on one thought for very long and may be easily distracted, unable to focus attention.

### • Emotional Expression

People with schizophrenia often show "blunted" or "flat" affect. This refers to a severe reduction in emotional expressiveness.

Some people have only one such psychotic episode; others have many episodes during a lifetime, but lead relatively normal lives during the interim periods. However, the individual with "chronic" schizophrenia, or a continuous or recurring pattern of illness, often does not fully recover normal functioning and typically requires long-term treatment, generally including medication, to control the symptoms.

There is no known single cause of schizophrenia. Many diseases, such as heart disease, result from an interplay of genetic, behavioral, and other factors; and this may be the case for schizophrenia as well. It has long been known that schizophrenia runs in families. Scientists are studying genetic factors in schizophrenia. It appears likely that multiple genes are involved in creating a predisposition to develop the disorder. In addition, factors such as prenatal difficulties like intrauterine starvation or viral infections, perinatal complications, and various nonspecific stressors, seem to influence the development of schizophrenia. However, it is not yet understood how the genetic predisposition is transmitted, and it cannot yet be accurately predicted whether a given person will or will not develop the disorder.

Basic knowledge about brain chemistry and its link to schizophrenia is expanding rapidly. It is likely, although not yet certain, that the disorder is associated with some imbalance of the complex, interrelated chemical systems of the brain, perhaps involving the neurotransmitters dopamine and glutamate.

Many studies of people with schizophrenia have found abnormalities in brain structure (for example, enlargement of the fluid-filled cavities, called the ventricles, in the interior of the brain, and decreased size of certain brain regions) or function (for example, decreased metabolic activity in certain brain regions). It should be emphasized that these abnormalities are quite subtle and are not characteristic of all people with schizophrenia, nor do they occur *only* in individuals with this illness. Microscopic studies of brain tissue after death have also shown small changes in distribution or number of brain cells in people with schizophrenia. It appears that many (but probably not all) of these changes are present before an individual becomes ill, and schizophrenia may be, in part, a disorder in development of the brain.

Available treatments can relieve many symptoms, but most people with schizophrenia continue to suffer some symptoms throughout their lives; it has been estimated that no more than one in five individuals recovers completely. Medications and other treatments for schizophrenia can help reduce and control the distressing symptoms of the illness. However, some people are not greatly helped by available treatments or may prematurely discontinue treatment because of unpleasant side effects. Since schizophrenia may not be a single condition and its causes are not yet known, current treatment methods are based on both clinical research and experience. These approaches are chosen on the basis of their ability to reduce the symptoms of schizophrenia and to lessen the chances that symptoms will return.

Antipsychotic medications have been available since the mid-1950s but they do not "cure" schizophrenia or ensure that there will be no further psychotic episodes. A number of new antipsychotic drugs (the so-called "atypical antipsychotics") have been introduced since 1990. The first of these, clozapine, has been shown to be more effective than other antipsychotics, although the possibility of severe side effects - in particular, a condition called agranulocytosis (loss of the white blood cells that fight infection) - requires that patients be monitored with blood tests every one or two weeks. Even newer antipsychotic drugs, such as risperidone and olanzapine are safer than the older drugs or clozapine, and they also may be better tolerated. They may or may not treat the illness as well as clozapine, however.

Antipsychotic drugs are often very effective in treating certain symptoms of schizophrenia, particularly hallucinations and delusions; unfortunately, the drugs may not be as helpful with other symptoms, such as reduced motivation and emotional expressiveness.

Current limitations of antipsychotic drug therapy include limited efficacy, side effects and delayed onset of action. Valproate was shown to enhance the onset of action of olanzapine or risperidone (Casey et al., Neuropsychopharmacology 28:182ff, 2003). Furthermore, another double-blind placebo controlled clinical trial demonstrated that lamotrigine, when added to clozapine, is beneficial for the treatment of treatment-resistant schizophrenia (Tihohen et al., Biol Psychiatr. 54:1241ff, 2003). It is estimated that currently up to 50% of hospitalized schizophrenic patients receive adjunct therapy with anti-epileptic drugs (Citrome L., Psychopharmacology Bull 37(S2):p74ff, 2003) especially for enhancing the onset of action, for controlling the positive symptoms, e.g. hallucinations, the affective symptoms, e.g. depresssion, as well as the cognitive symptoms, e.g. attention deficit.

The use of compounds of Formula (Ib) or/and Formula (IIb) for treatment of schizophrenia in an add-on therapy has not been reported. Thus, the present invention concerns the use of said compound(s) of Formulae (Ib) or/and (IIb) for the preparation of a pharmaceutical composition for the prevention, alleviation or/and treatment of schizophrenia in an add-on therapy.

The application of SPM 927 alone had no significant effect on prepulse inhibition in mice, which is a model of predicitive validity for schizophrenia. Surprisingly, the compounds (Ib) or/and (IIb), particularly (R)-2-acetamide-N-benzyl-3-methoxypropionamide (SPM 927) potentiated the increase of prepulse inhibition of an antipsychotic drug, particularly clozapine, when administered together.

An add-on therapy according to the present invention for the prevention, alleviation or/and treatment of schizophrenia is the co-administration of at least one compound of Formulae (Ib) or/and (IIb) with at least one agent useful for the prevention, alleviation or/and treatment of schizophrenia, e.g. antipsychotic agent(s), in order to increase the efficacy, decrease the side effects or/and enhance the onset of action of the antipsychotic medication, for example.

Co-administration comprises administration of the agents in amounts sufficient to achieve or/and maintain therapeutically effective concentrations, e.g. plasma concentrations, in the subject in need thereof. Co-administration comprises simultaneous or/and subsequent administration. Simultaneous administration comprises administration of the agents as a single or as different compositions (see below) "at the same time" within the treatment period. Subsequent administration comprises administration of the agents "at intervals" within the treatment period.

Administration "at the same time" includes administration of the agent(s) useful for the prevention, alleviation or/and treatment of schizophrenia and the compound(s) of Formulae (Ib) or/and (IIb) literally "at the same time", but also includes administration directly one after another. Administration "at intervals" includes administration of the agent(s) useful for the prevention, alleviation or/and treatment of schizophrenia and the compound(s) of Formulae (Ib) or/and (IIb) at an interval of 1 h at the maximum, preferably 6 h at the maximum, more preferably 12 h at the maximum, even more preferably 1 day at the maximum, and most preferably 1 month at the maximum.

The agent(s) useful for the prevention, alleviation or/and treatment of schizophrenia and the at least one compound of Formulae (Ib) or/and (IIb) may be formulated in one pharmaceutical preparation (single dose form) for administration at the same time or may be formulated in two distinct preparations (separate dose form) for administration at the same time or at intervals, as described in the preferred embodiments below for example. The two distinct preparations in the separate dose form may be administered by the same route or by different routes.

Depending on the dosage forms, which may be identical or different, e.g. fast release dosage forms, controlled release dosage forms or/and depot forms, the agent(s) useful for the prevention, alleviation or/and treatment of schizophrenia and the compound(s) of Formulae (Ib) or/and (IIb) may be administered with the same or with different schedules on a daily, weekly or monthly basis. Therefore, the administration interval of the agent(s) useful for the prevention, alleviation or/and treatment of schizophrenia and the compound(s) of Formulae (Ib) or/and (IIb) may depend on the administration schedules or/and on the dosage forms.

In a preferred embodiment, the compounds of Formulae (Ib) or/and (IIb) are used for the preparation of a pharmaceutical composition comprising
(a) at least one agent useful for the prevention, alleviation or/and treatment of schizophrenia, and
(b) at least one compound of Formulae (Ib) or/and (IIb).

The at least one agent useful for the prevention, alleviation or treatment of schizophrenia may be an antipsychotic agent, more preferably an atypical antipsychotic agent, and most preferably the agent is clozapine, risperidone, aripiprazole, quietiapine or/and olanzapine.

In a further preferred embodiment, the compounds of Formulae (Ib) or/and (IIb) are used for the preparation of a pharmaceutical composition comprising a single dose form comprising at least one compound according to Formula (Ib) or/and Formula (IIb) and at least one agent useful for the prevention, alleviation or/and treatment of schizophrenia in the form of one composition.

In another preferred embodiment, the compounds of Formulae (Ib) or/and (IIb) are used for the preparation of a pharmaceutical composition which is a a separate dose form comprising
(i) a first composition comprising at least one agent useful for the prevention, alleviation or/and treatment of schizophrenia, and
(ii) a second composition comprising at least one compound of Formulae (Ib) or/and (IIb).

In yet another preferred embodiment of the present invention, a commercially available composition of an agent useful for prevention, alleviation or/and treatment of schizophrenia may be administered to a subject in need thereof. Therefore, in this preferred embodiment, the compounds of Formulae (Ib) or/and (IIb) are used for the preparation of a pharmaceutical composition comprising at least one compound according to Formula (Ib) or/and Formula (IIb) and not comprising an agent useful for the prevention, alleviation or/and treatment schizophrenia, e.g. an antipsychotic agent.

The use according to the present invention may comprise the preparation of a pharmaceutical compostion for administration of the at least one compound of Formulae (Ib) or/and (IIb) and the at least one agent useful for the prevention, alleviation or/and treatment of schizophrenia at the same time or at intervals, as defined above.

A compound according to the invention has the general Formula (Ib) 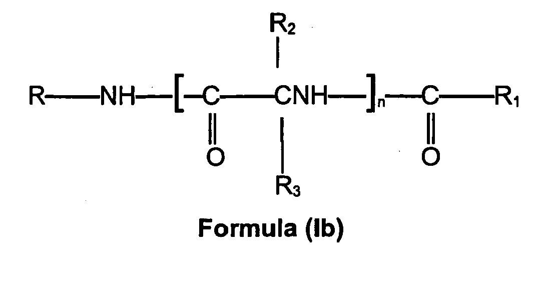 wherein
R is hydrogen, lower alkyl, lower alkenyl, lower alkynyl, aryl, aryl lower alkyl, heterocyclic, heterocyclic lower alkyl, lower alkyl heterocyclic, lower cycloalkyl or lower cycloalkyl lower alkyl, and R is unsubstituted or is substituted with at least one electron withdrawing group, and/or at least one electron donating group;
R₁ is hydrogen or lower alkyl, lower alkenyl, lower alkynyl, aryl lower alkyl, aryl, heterocyclic lower alkyl, lower alkyl heterocyclic, heterocyclic, lower cycloalkyl, lower cycloalkyl lower alkyl, each unsubstituted or substituted with at least one electron donating group and/or at least one electron withdrawing group;
and
R₂ and R₃ are independently hydrogen, lower alkyl, lower alkenyl, lower alkynyl, aryl lower alkyl, aryl, halo, heterocyclic, heterocyclic lower alkyl, lower alkyl heterocyclic, lower cycloalkyl, lower cycloalkyl lower alkyl, or Z-Y wherein R₂ and R₃ may be unsubstituted or substituted with at least one electron withdrawing group and/or at least one electron donating group;
Z is O, S, S(O)ₐ, NR₄, NR'₆, PR₄ or a chemical bond;
Y is hydrogen, lower alkyl, aryl, aryl lower alkyl, lower alkenyl, lower alkynyl, halo, heterocyclic, heterocyclic lower alkyl, lower alkyl heterocyclic and Y may be unsubstituted or substituted with at least one electron donating group and/or at least one electron withdrawing group, provided that when Y is halo, Z is a chemical bond, or
ZY taken together is NR₄NR₅R₇, NR₄OR₅, ONR₄R₇, OPR₄R₅, PR₄OR₅, SNR₄R₇, NR₄SR₇, SPR₄R₅, PR₄SR₇, NR₄PR₅R₆, PR₄NR₅R₇ or N⁺R₅R₆R₇, 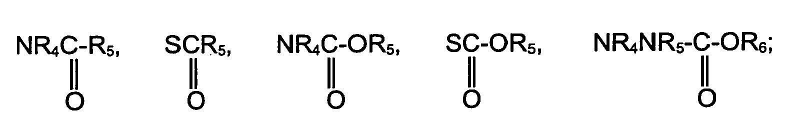 R'₆ is hydrogen, lower alkyl, lower alkenyl, or lower alkenyl which may be unsubstituted or substituted with at least one electron withdrawing group or/and at least one electron donating group;
R₄, R₅ and R₆ are independently hydrogen, lower alkyl, aryl, aryl lower alkyl, lower alkenyl, or lower alkynyl, wherein R₄, R₅ and R₆ may independently be unsubstituted or substituted with at least one electron withdrawing group or/and at least one electron donating group;
R₇ is R₆ or COOR₈ or COR₈, which R₇ may be unsubstituted or substituted with at least one electron withdrawing group or/and at least one electron donating group;
R₈ is hydrogen or lower alkyl, or aryl lower alkyl, and the aryl or alkyl group may be unsubstituted or substituted with at least one electron withdrawing group or/and at least one electron donating group; and
n is 1-4; and
a is 1-3.

Preferably the compound according has the general Formula (IIb) 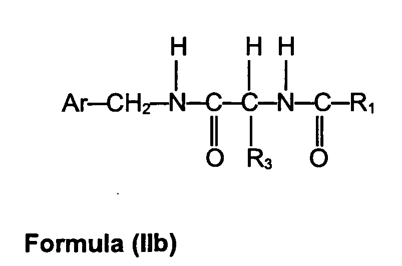 wherein
Ar is aryl, especially phenyl, which is unsubstituted or substituted with at least one halo; R₃ is -CH₂-Q, wherein Q is lower alkoxy; and R₁ is lower alkyl, especially methyl.

The present invention is also directed to a pharmaceutical composition comprising at least one compound according to Formula (Ib) or/and Formula (IIb) useful for the prevention, alleviation or/and treatment of schizophrenia in an add-on therapy.

In a preferred embodiment, the pharmaceutical composition comprises
(a) at least one agent useful for the prevention, alleviation or/and treatment of schizophrenia, and
(b) at least one compound of Formulae (Ib) or/and (IIb).

The at least one agent useful for the prevention, alleviation or treatment of schizophrenia may be an antipsychotic agent, more preferably an atypical antipsychotic agent, and most preferably the agent is clozapine, risperidone, aripiprazole, quietiapine or/and olanzapine.

In a further preferred embodiment, the pharmaceutical composition of the present invention comprises a single dose form comprising at least one compound according to Formula (Ib) or/and Formula (IIb) and at least one agent useful for the prevention, alleviation or/and treatment of schizophrenia in the form of one composition.

In another preferred embodiment, the pharmaceutical composition of the present invention comprises a separate dose form comprising
(i) a first composition comprising at least one agent useful for the prevention, alleviation or/and treatment of schizophrenia, and
(ii) a second composition comprising at least one compound of Formulae (Ib) or/and (IIb).

In yet another preferred embodiment of the present invention, a commercially available composition of an agent useful for the prevention, alleviation or/and treatment of schizophrenia may be administered to a subject in need thereof. Therefore, in this preferred embodiment, the pharmaceutical composition of the present invention comprises at least one compound according to Formula (Ib) or/and Formula (IIb) and does not comprise an agent useful for the prevention, alleviation or/and treatment of schizophrenia, e.g. an antipsychotic agent.

The pharmaceutical composition of the present invention may be prepared for administration of the at least one compound of Formulae (Ib) or/and (IIb) and the at least one agent useful for the prevention, alleviation or/and treatment of schizophrenia at the same time or at intervals, as defined above.

The "lower alkyl" groups when used alone or in combination with other groups, are lower alkyl containing from 1 to 6 carbon atoms, especially 1 to 3 carbon atoms, and may be straight chain or branched. These groups include methyl, ethyl, propyl, isopropyl, butyl, isobutyl, tertiary butyl, amyl, hexyl, and the like.

The "lower alkoxy" groups are lower alkoxy containing from 1 to 6 carbon atoms, especially 1 to 3 carbon atoms, and may be straight chain or branched. These groups include methoxy, ethoxy, propoxy, butoxy, isobutoxy, tert-butoxy, pentoxy, hexoxy and the like.

The "aryl lower alkyl" groups include, for example, benzyl, phenylethyl, phenylpropyl, phenylisopropyl, phenylbutyl, diphenylmethyl, 1,1-diphenylethyl, 1,2-diphenylethyl, and the like.

The term "aryl", when used alone or in combination, refers to an aromatic group which contains from 6 up to 18 ring carbon atoms and up to a total of 25 carbon atoms and includes the polynuclear aromatics. These aryl groups may be monocyclic, bicyclic, tricyclic or polycyclic and are fused rings. A polynuclear aromatic compound as used herein, is meant to encompass bicyclic and tricyclic fused aromatic ring systems containing from 10-18 ring carbon atoms and up to a total of 25 carbon atoms. The aryl group includes phenyl, and the polynuclear aromatics e.g., naphthyl, anthracenyl, phenanthrenyl, azulenyl and the like. The aryl group also includes groups like ferrocenyl. Aryl groups may be unsubstituted or mono or polysubstituted with electron withdrawing or/and electron donating groups as described below.

"Lower alkenyl" is an alkenyl group containing from 2 to 6 carbon atoms and at least one double bond. These groups may be straight chained or branched and may be in the Z or E form. Such groups include vinyl, propenyl, 1-butenyl, isobutenyl, 2-butenyl, 1-pentenyl, (Z)-2-pentenyl, (E)-2-pentenyl, (Z)-4-methyl-2-pentenyl, (E)-4-methyl-2-pentenyl, pentadienyl, e.g., 1, 3 or 2,4-pentadienyl, and the like.

The term "lower alkynyl" is an alkynyl group containing 2 to 6 carbon atoms and may be straight chained as well as branched. It includes such groups as ethynyl, propynyl, 1-butynyl, 2-butynyl, 1-pentynyl, 2-pentynyl, 3-methyl-1-pentynyl, 3-pentynyl, 1-hexynyl, 2-hexynyl, 3-hexynyl and the like.

The term "lower cycloalkyl" when used alone or in combination is a cycloalkyl group containing from 3 to 18 ring carbon atoms and up to a total of 25 carbon atoms. The cycloalkyl groups may be monocyclic, bicyclic, tricyclic, or polycyclic and the rings are fused. The cycloalkyl may be completely saturated or partially saturated. Examples include cyclopropyl, cyclobutyl, cyclopentyl, cyclohexyl, cycloheptyl, cyclooctyl, cyclodecyl, cyclohexenyl, cyclopentenyl, cyclooctenyl, cycloheptenyl, decalinyl, hydroindanyl, indanyl, fenchyl, pinenyl, adamantyl, and the like. Cycloalkyl includes the cis or trans forms. Cycloalkyl groups may be unsubstituted or mono or polysubstituted with electron withdrawing or/and electron donating groups as described below. Furthermore, the substituents may either be in endo or exo positions in the bridged bicyclic systems.

The term "electron-withdrawing and electron donating" refer to the ability of a substituent to withdraw or donate electrons, respectively, relative to that of hydrogen if the hydrogen atom occupied the same position in the molecule. These terms are well understood by one skilled in the art and are discussed in Advanced Organic Chemistry, by J. March, John Wiley and Sons, New York, NY, pp.16-18 (1985) and the discussion therein is incorporated herein by reference. Electron withdrawing groups include halo, including bromo, fluoro, chloro, iodo and the like; nitro, carboxy, lower alkenyl, lower alkynyl, formyl, carboxyamido, aryl, quaternary ammonium, haloalkyl such as trifluoromethyl, aryl lower alkanoyl, carbalkoxy and the like. Electron donating groups include such groups as hydroxy, lower alkoxy, including methoxy, ethoxy and the like; lower alkyl, such as methyl, ethyl, and the like; amino, lower alkylamino, di(loweralkyl) amino, aryloxy such as phenoxy, mercapto, lower alkylthio, lower alkylmercapto, disulfide (lower alkyldithio) and the like. One of ordinary skill in the art will appreciate that some of the aforesaid substituents may be considered to be electron donating or electron withdrawing under different chemical conditions. Moreover, the present invention contemplates any combination of substituents selected from the above-identified groups.

The term "halo" includes fluoro, chloro, bromo, iodo and the like.

The term "acyl" includes lower alkanoyl containing from 1 to 6 carbon atoms and may be straight chains or branched. These groups include, for example, formyl, acetyl, propionyl, butyryl, isobutyryl, tertiary butyryl, pentanoyl and hexanoyl.

As employed herein, a heterocyclic group contains at least one sulfur, nitrogen or oxygen ring atom, but also may include several of said atoms in the ring. The heterocyclic groups contemplated by the present invention include heteroaromatics and saturated and partially saturated heterocyclic compounds. These heterocyclics may be monocyclic, bicyclic, tricyclic or polycyclic and are fused rings. They may preferably contain up to 18 ring atoms and up to a total of 17 ring carbon atoms and a total of up to 25 carbon atoms. The heterocyclics are also intended to include the so-called benzoheterocyclics. Representative heterocyclics include furyl, thienyl, pyrazolyl, pyrrolyl, methylpyrrolyl, imidazolyl, indolyl, thiazolyl, oxazolyl, isothiazolyl, isoxazolyl, piperidyl, pyrrolinyl, piperazinyl, quinolyl, triazolyl, tetrazolyl, isoquinolyl, benzofuryl, benzothienyl, morpholinyl, benzoxazolyl, tetrahydrofuryl, pyranyl, indazolyl, purinyl, indolinyl, pyrazolindinyl, imidazolinyl, imadazolindinyl, pyrrolidinyl, furazanyl, N-methylindolyl, methylfuryl, pyridazinyl, pyrimidinyl, pyrazinyl, pyridyl, epoxy, aziridino, oxetanyl, azetidinyl, the N-oxides of the nitrogen containing heterocycles, such as the N-oxides of pyridyl, pyrazinyl, and pyrimidinyl and the like. Heterocyclic groups may be unsubstituted or mono or polysubstituted with electron withdrawing or/and electron donating groups.

The preferred heterocyclics are thienyl, furyl, pyrrolyl, benzofuryl, benzothienyl, indolyl, methylpyrrolyl, morpholinyl, pyridiyl, pyrazinyl, imidazolyl, pyrimidinyl, or pyridazinyl. The preferred heterocyclic is a 5 or 6-membered heterocyclic compound. The especially preferred heterocyclic is furyl, pyridyl, pyrazinyl, imidazolyl, pyrimidinyl, or pyridazinyl. The most preferred heterocyclics are furyl and pyridyl.

The preferred compounds are those wherein n is 1, but di (n=2), tri (n=3) and tetrapeptides (n=4) are also contemplated to be within the scope of the invention.

The preferred values of R is aryl lower alkyl, especially benzyl especially those wherein the phenyl ring thereof is unsubstituted or substituted with electron donating groups or/and electron withdrawing groups, such as halo (e.g., F).

The preferred R₁ is H or lower alkyl. The most preferred R₁ group is methyl.

The preferred electron donating substituents or/and electron withdrawing substituents are halo, nitro, alkanoyl, formyl, arylalkanoyl, aryloyl, carboxyl, carbalkoxy, carboxamido, cyano, sulfonyl, sulfoxide, heterocyclic, guanidine, quaternary ammonium, lower alkenyl, lower alkynyl, sulfonium salts, hydroxy, lower alkoxy, lower alkyl, amino, lower alkylamino, di(loweralkyl) amino, amino lower alkyl, mercapto, mercaptoalkyl, alkylthio, and alkyldithio. The term "sulfide" encompasses mercapto, mercapto alkyl and alkylthio, while the term disulfide encompasses alkyldithio. Especially preferred electron donating or/and electron withdrawing groups are halo or lower alkoxy, most preferred are fluoro or methoxy. These preferred substituents may be present on any one of the groups in Formula (lb) or/and (llb), e.g. R, R₁, R₂, R₃, R₄, R₅ R₆, R'₆, R₇ and/or R₈as defined herein.

The ZY groups representative of R₂ and R₃ include hydroxy, alkoxy, such as methoxy, ethoxy, aryloxy, such as phenoxy; thioalkoxy, such as thiomethoxy, thioethoxy; thioaryloxy such as thiophenoxy; amino; alkylamino, such as methylamino, ethylamino; arylamino, such as anilino; lower dialkylamino, such as, dimethylamino; trialkyl ammonium salt, hydrazino; alkylhydrazino and arylhydrazino, such as N-methylhydrazino, N-phenylhydrazino, carbalkoxy hydrazino, aralkoxycarbonyl hydrazino, aryloxycarbonyl hydrazino, hydroxylamino, such as N-hydroxylamino (-NH-OH), lower alkoxy amino [(NHOR₁₈) wherein R₁₈ is lower alkyl], N-lower alkylhydroxyl amino [(NR₁₈)OH wherein R₁₈ is lower alkyl], N-lower alkyl-O-lower alkylhydroxyamino, i.e., [N(R₁₈)OR₁₉ wherein R₁₈ and R₁₉ are independently lower alkyl], and o-hydroxylamino (-O-NH₂); alkylamido such as acetamido; trifluoroacetamido; lower alkoxyamino, (e.g., NH(OCH₃); and heterocyclicamino, such as pyrazoylamino.

The preferred heterocyclic groups representative of R₂ and R₃ are monocyclic 5- or 6-membered heterocyclic moieties of the formula: 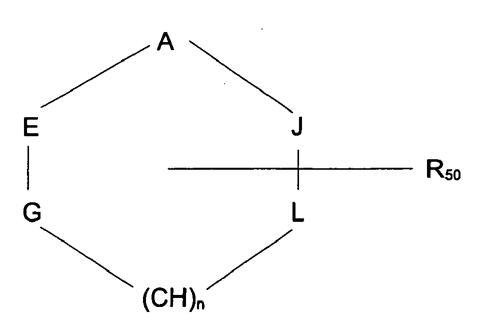 or those corresponding partially or fully saturated form thereof wherein n is 0 or 1; and
R₅₀ is H or an electron withdrawing group or electron donating group;
A, E, L, J and G are independently CH, or a heteroatom selected from the group consisting of N, O, S;
but when n is 0, G is CH, or a heteroatom selected from the group consisting of NH, O and S with the proviso that at most two of A, E, L, J and G are heteroatoms.

When n is 0, the above heteroaromatic moiety is a five membered ring, while if n is 1, the heterocyclic moiety is a six membered monocyclic heterocyclic moiety. The preferred heterocyclic moieties are those aforementioned heterocyclics which are monocyclic.

If the ring depicted hereinabove contains a nitrogen ring atom, then the N-oxide forms are also contemplated to be within the scope of the invention.

When R₂ or R₃ is a heterocyclic of the above formula, it may be bonded to the main chain by a ring carbon atom. When n is 0, R₂ or R₃ may additionally be bonded to the main chain by a nitrogen ring atom.

Other preferred moieties of R₂ and R₃ are hydrogen, aryl, e.g., phenyl, aryl alkyl, e.g., benzyl and alkyl.

It is to be understood that the preferred groups of R₂ and R₃ may be unsubstituted or mono or poly substituted with electron donating or/and electron withdrawing groups. It is preferred that R₂ and R₃ are independently hydrogen, lower alkyl, which is either unsubstituted or substituted with electron withdrawing groups or/and electron donating groups, such as lower alkoxy (e.g., methoxy, ethoxy, and the like), N-hydroxylamino, N-lower alkylhydroxyamino, N-loweralkyl-O-loweralkyl and alkylhydroxyamino.

It is preferred that one of R₂ and R₃ is hydrogen.

It is preferred that n is one.

It is more prefered that n=1 and one of R₂ and R₃ is hydrogen. It is especially preferred that in this embodiment, R₂ is hydrogen and R₃ is lower alkyl or ZY; Z is O, NR₄ or PR₄; Y is hydrogen or lower alkyl; ZY is NR₄NR₅R₇, NR₄OR₅, ONR₄R₇,

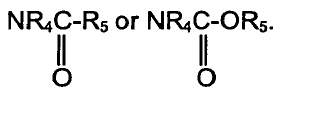

In another especially preferred embodiment, n=1, R₂ is hydrogen and R₃ is lower alkyl which may be substituted or unsubstituted with an electron donating or electron withdrawing group, NR₄OR₅, or ONR₄R_{7,}

In yet another especially preferred embodiment, n = 1, R₂ is hydrogen and R₃ is lower alkyl which is unsubstituted or substituted with hydroxy or loweralkoxy, NR₄OR₅ or ONR₄R₇, wherein R₄, R₅ and R₇ are independently hydrogen or lower alkyl, R is aryl lower alkyl, which aryl group may be unsubstituted or substituted with an electron withdrawing group and R₁ is lower alkyl. In this embodiment it is most preferred that aryl is phenyl, which is unsubstituted or substituted with halo.

It is preferred that R₂ is hydrogen and R₃ is hydrogen, an alkyl group which is unsubstituted or substituted by at least an electron donating or electron withdrawing group or ZY. In this preferred embodiment, it is more preferred that R₃ is hydrogen, an alkyl group such as methyl, which is unsubstituted or substituted by an electron donating group, or NR₄OR₅ or ONR₄R₇, wherein R₄, R₅ and R₇ are independently hydrogen or lower alkyl. It is preferred that the electron donating group is lower alkoxy, and especially methoxy or ethoxy.

It is preferred that R₂ and R₃ are independently hydrogen, lower alkyl, or ZY;
Z is O, NR₄ or PR₄;
Y is hydrogen or lower alkyl or
ZY is NR₄R₅R₇, NR₄OR₅, ONR₄R₇,

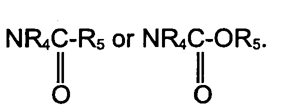

It is also preferred that R is aryl lower alkyl. The most preferred aryl for R is phenyl. The most preferred R group is benzyl. In a preferred embodiment, the aryl group may be unsubstituted or substituted with an electron donating or electron withdrawing group. If the aryl ring in R is substituted, it is most preferred that it is substituted with an electron withdrawing group, especially on the aryl ring. The most preferred electron withdrawing group for R is halo, especially fluoro.

The preferred R₁ is lower alkyl, especially methyl.

It is more preferred that R is aryl lower alkyl and R₁ is lower alkyl.

Further preferred compounds are compounds of Formula (Ib) wherein n is 1; R₂ is hydrogen; R₃ is hydrogen, a lower alkyl group, especially methyl which is substituted by an electron donating or electron withdrawing group or ZY; R is aryl, aryl lower alkyl, such as benzyl, wherein the aryl group is unsubstituted or substituted with an electron donating or electron withdrawing group and R₁ is lower alkyl. In this embodiment, it is more preferred that R₃ is hydrogen, a lower alkyl group, especially methyl, which may be substituted by electron donating group, such as lower alkoxy, (e.g., methoxy, ethoxy and the like), NR₄OR₅ or ONR₄R₇ wherein these groups are defined hereinabove.

The most preferred compounds utilized are those of the Formula (IIb): 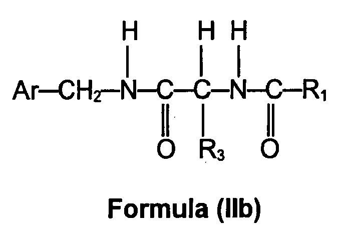 wherein
Ar is aryl, especially phenyl, which is unsubstituted or substituted with at least one electron donating group or electron withdrawing group, especially halo,
R₁ is lower alkyl, especially containing 1-3 carbon atoms; and
R₃ is as defined herein, but especially hydrogen, loweralkyl, which is unsubstituted or substituted by at least an electron donating group or electron withdrawing group or ZY. It is even more preferred that R₃ is, in this embodiment, hydrogen, an alkyl group which is unsubstituted or substituted by an electron donating group, NR₄OR₅ or ONR₄R₇. It is most preferred that R₃ is CH₂-Q, wherein Q is lower alkoxy, especially containing 1-3 carbon atoms; NR₄OR₅ or ONR₄R₇ wherein R₄ is hydrogen or alkyl containing 1-3 carbon atoms, R₅ is hydrogen or alkyl containing 1-3 carbon atoms, and R₇ is hydrogen or alkyl containing 1-3 carbon atoms.

The most preferred R₁ is CH₃. The most preferred R₃ is CH₂-Q, wherein Q is methoxy.

The most preferred aryl is phenyl. The most preferred halo is fluoro.

The most preferred compounds include:
(R)-2-acetamido-N-benzyl-3-methoxy-propionamide;
O-methyl-N-acetyl-D-serine-m-fluorobenzyl-amide;
O-methyl-N-acetyl-D-serine-p-fluorobenzyl-amide;
N-acetyl-D-phenylglycine benzylamide;
D-1,2-(N,O-dimethylhydroxylamino)-2-acetamide acetic acid benzylamide;
D-1,2-(O-methylhydroxylamino)-2-acetamido acetic acid benzylamide.

It is to be understood that the various combinations and permutations of the Markush groups of R₁, R₂, R₃, R and n described herein are contemplated to be within the scope of the present invention. Moreover, the present invention also encompasses compounds and compositions which contain one or more elements of each of the Markush groupings in R₁, R₂, R₃, n and R and the various combinations thereof. Thus, for example, the present invention contemplates that R₁ may be one or more of the substituents listed hereinabove in combination with any and all of the substituents of R₂, R₃, and R with respect to each value of n.

The compounds utilized in the present invention may contain one or more asymmetric carbons and may exist in racemic and optically active forms. The configuration around each asymmetric carbon can be either the D or L form. It is well known in the art that the configuration around a chiral carbon atoms can also be described as R or S in the Cahn-Prelog-Ingold nomenclature system. All of the various configurations around each asymmetric carbon, including the various enantiomers and diastereomers as well as racemic mixtures and mixtures of enantiomers, diastereomers or both are contemplated by the present invention.

In the principal chain, there exists asymmetry at the carbon atom to which the groups R₂ and R₃ are attached. When n is 1, the compounds of the present invention is of the formula 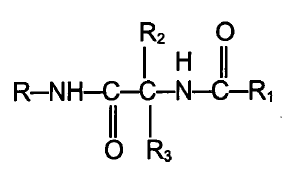 wherein R, R₁, R₂, R₃, R₄, R₅, R₆, R'₆, R₇, R₈, R₅₀ Z and Y are as defined previously.

As used herein, the term configuration shall refer to the configuration around the carbon atom to which R₂ and R₃ are attached, even though other chiral centers may be present in the molecule. Therefore, when referring to a particular configuration, such as D or L, it is to be understood to mean the D or L stereoisomer at the carbon atom to which R₂ and R₃ are attached. However, it also includes all possible enantiomers and diastereomers at other chiral centers, if any, present in the compound.

The compounds of the present invention are directed to all the optical isomers, i.e., the compounds of the present invention are either the L-stereoisomer or the D-stereoisomer (at the carbon atom to which R₂ and R₃ are attached). These stereoisomers may be found in mixtures of the L and D stereoisomer, e.g., racemic mixtures. The D stereoisomer is preferred.

Depending upon the substituents, the present compounds may form addition salts as well. All of these forms are contemplated to be within the scope of this invention including mixtures of the stereoisomeric forms.

The manufacture of the utilized compounds is described in U.S. Patent Nos. 5,378,729 and 5,773.475, the contents of both of which are incorporated by reference.

The compounds utilized in the present invention are useful as such as depicted in the Formulae (Ib) or/and (IIb) or can be employed in the form of salts in view of its basic nature by the presence of the free amino group.

Thus, the compounds of Formulae (Ib) or/and (IIb) forms salts with a wide variety of acids, inorganic and organic, including pharmaceutically acceptable acids. The salts with therapeutically acceptable acids are of course useful in the preparation of formulation where enhanced water solubility is most advantageous.

These pharmaceutically acceptable salts have also therapeutic efficacy. These salts include salts of inorganic acids such as hydrochloric, hydroiodic, hydrobromic, phosphoric, metaphosphoric, nitric acid and sulfuric acids as well as salts of organic acids, such as tartaric, acetic, citric, malic, benzoic, perchloric, glycolic, gluconic, succinic, aryl sulfonic, (e.g., p-toluene sulfonic acids, benzenesulfonic), phosphoric, malonic, and the like.

The present invention is further directed to a method for the prevention, alleviation or/and treatment of schizophrenia in an add-on therapy by administration of at least one compound of Formulae (Ib) or/and (IIb) to a subject in need thereof.

In a preferred embodiment, the method of the present invention comprises
(a) the administration of at least one agent useful for the prevention, alleviation or/and treatment of schizophrenia, and
(b) the administration of at least one compound of Formulae (Ib) or/and (IIb) in an add-on therapy in addition to the at least one agent of (a),
to a subject in need thereof.

In the method of the present invention, the at least one agent useful for the prevention, alleviation or treatment of schizophrenia may be an antipsychotic agent, more preferably an atypical antipsychotic agent, and most preferably the agent is clozapine, risperidone, aripiprazole, quietiapine or/and olanzapine.

It is further preferred that the at least one compound of Formulae (Ib) or/and (IIb) and the at least one agent useful for the prevention, alleviation or/and treatment of schizophrenia are administered in the form of one composition (single dose form).

In another preferred embodiment, the method of the present invention comprises the administration of a separate dose form comprising
(i) a first composition comprising at least one agent useful for the prevention, alleviation or/and treatment of schizophrenia, and
(ii) a second composition comprising at least one compound of Formulae (Ib) or/and (IIb).
to a subject in need thereof.

In yet another preferred embodiment of the present invention, a commercially available composition of an agent useful for prevention, alleviation or/and treatment of schizophrenia may be administered to a subject in need thereof. Therefore, in this preferred embodiment, the method of the present invention for prevention, alleviation or/and treatment of schizophrenia comprises administration of a pharmaceutical composition comprising at least one compound according to Formula (Ib) or/and Formula (IIb) and not comprising an agent useful for the prevention, alleviation or/and treatment of schizophrenia, e.g. an antipsychotic agent, to a subject receiving a commercially available composition for prevention, alleviation or/and treatment of schizophrenia, e.g. an antipsychotic agent.

The compounds according to Formulae (Ib) or/and (IIb) may also be useful in an add-on therapy for disorders or/and diseases other than schizophrenia which are treated by antipsychotic agents. Therefore, yet another subject of the present invention is an add-on therapy for the prevention, alleviation or/and treatment of bipolar disorder, autism, psychosis in diseases other than schizophrenia, e.g. in Alzheimer's disease, or/and attention deficit hyperactivity disorder, by administration of a pharmaceutical composition of the present invention. The compounds according to Formulae (Ib) or/and (IIb) may also be used for the preparation of a pharmaceutical composition useful for the prevention, alleviation or/and treatment in an add-on therapy for disorders or/and diseases other than schizophrenia which can be treated by antipsychotic agents, such as bipolar disorder, autism, psychosis in diseases other than schizophrenia, e.g. in Alzheimer's disease, or/and attention deficit hyperactivity disorder. The pharmaceutical composition of the present invention as described herein for treatment of schizophrenia may also be used for the prevention, alleviation or/and treatment in an add-on therapy for disorders or/and diseases other than schizophrenia which can be treated by antipsychotic agents, such as bipolar disorder, autism, psychosis in diseases other than schizophrenia, e.g. in Alzheimer's disease, or/and attention deficit hyperactivity disorder.

It is preferred that the compound utilized in the present invention is used in therapeutically effective amounts.

The physician will determine the dosage of the present therapeutic agents which will be most suitable and it will vary with the form of administration and the particular compound chosen, and furthermore, it will vary with the patient under treatment, the age of the patient, the type of malady being treated. He will generally wish to initiate treatment with small dosages substantially less than the optimum dose of the compound and increase the dosage by small increments until the optimum effect under the circumstances is reached. When the composition is administered orally, larger quantities of the active agent will be required to produce the same effect as a smaller quantity given parenterally. The compounds are useful in the same manner as comparable therapeutic agents and the dosage level is of the same order of magnitude as is generally employed with these other therapeutic agents.

Typical doses of antipsychotic agents administered to a subject in need thereof in the add-on therapy of the present invention are: olanzapine 5-20 mg/day, clozapine 100-900 mg/day, quietiapine 100-800 mg/day, risperidone 1-16 mg/day, or/and aripiprazole 3-30 mg/day. Such doses may also be included in the pharmaceutical composition of the present invention, or/and may be used for the preparation of a pharmaceutical composition of the present invention.

The physician is able to determine the administration route of the at least one agent useful for the prevention, alleviation or/and treatment of schizophrenia, e.g. an antipsychotic agent, which may be the same or different to the administration route of the at least one compound of the present invention of Formulae (Ib) or/and (IIb). Typical administration routes of the at least one agent useful for the prevention, alleviation or/and treatment of schizophrenia are oral, intravenous, intramuscular, intrathecal or subcutaneous routes. Oral or/and intravenous administration is preferred.

In a preferred embodiment, the compounds of formulae (Ib) or/and (IIb) of the present invention are administered in amounts ranging from about 1 mg to about 10 mg per kilogram of body weight per day. This dosage regimen may be adjusted by the physician to provide the optimum therapeutic response. Patients in need thereof may be treated with doses of the compound of the present invention of at least 50 mg/day, preferably of at least 200 mg/day, more preferably of at least 300 mg/day and most preferably of at least 400 mg/day. Generally, a patient in need thereof may be treated with doses at a maximum of 6 g/day, more preferably a maximum of 1 g/day and most preferably a maximum of 600 mg/day. In some cases, however, higher or lower doses may be needed.

In another preferred embodiment, the daily doses are increased until a predetermined daily dose is reached which is maintained during the further treatment.

In yet another preferred embodiment, several divided doses may be administered daily. For example, three doses per day may be administered, preferably two doses per day. It is more preferred to administer a single dose per day.

In yet another preferred embodiment, an amount of the compounds of the present invention may be administered which results in a plasma concentration of 0.1 to 15 µg/ml (trough) and 5 to 18.5 µg/ml (peak), calculated as an average over a plurality of treated subjects.

The compounds of Formulae (Ib) or/and (IIb) may be administered in a convenient manner, such as by oral, intravenous (where water soluble), intramuscular, intrathecal or subcutaneous routes. Oral or/and i.v. administration is preferred.

The pharmaceutical composition of the present invention may be prepared for the treatment regimen as described above, in particular for the treatment with doses as described above, to effect plasma concentrations as described above, for administration periods or/and administration routes as specified in the embodiments of the present invention as described above.

The pharmaceutical compositions of the present invention comprising the at least one agent for the prevention, alleviation or/and treatment of schizophrenia is formulated with common carriers, diluents or/and auxiliary substances known to a person skilled in the art. In the case of a separate dose form, the first composition comprising at least one agent useful for the prevention, alleviation or/and treatment of schizophrenia and the second composition comprising at least one compound of Formulae (Ib) or/and (IIb) may comprise carriers, diluents or/and auxiliary substances which are independent from each other, identical or different in the first composition comprising at least one agent useful for the prevention, alleviation or/and treatment of schizophrenia and the second composition comprising at least one compound of Formulae (Ib) or/and (IIb).

The compounds of Formulae (Ib) or/and (IIb) may be orally administered, for example, with an inert diluent or with an assimilable edible carrier, or it may be enclosed in hard or soft shell gelatin capsules, or it may be compressed into tablets, or it may be incorporated directly into the fool of the diet. For oral therapeutic administration, the active compound of Formulae (Ib) or/and (IIb) may be incorporated with excipients and used in the form of ingestible tablets, buccal tablets, troches, capsules, elixirs, suspensions, syrups, wafers, and the like. Such compositions and preparations should contain at least 1 % of active compound of Formulae (Ib) or/and (IIb). The percentage of the compositions and preparations may, of course, be varied and may conveniently be between about 5 to about 80 % of the weight of the unit. The amount of active compound of Formulae (Ib) or/and (IIb) in such therapeutically useful compositions is such that a suitable dosage will be obtained. Preferred compositions or preparations according to the present invention contains between about 10 mg and 6 g active compound of Formulae (Ib) or/and (IIb).

The tablets, troches, pills, capsules and the like may also contain the following: A binder such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavoring agent such as peppermint, oil of wintergreen, or cherry flavoring. When the dosage unit form is a capsule, it may contain, in addition to materials of the above type, a liquid carrier.

Various other materials may be present as coatings or otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavoring such as cherry or orange flavor. Of course, any material used in preparing any dosage unit form should be pharmaceutically pure and substantially non-toxic in the amounts employed. In addition, the active compound may be incorporated into sustained-release preparations and formulations. For example, sustained release dosage forms are contemplated wherein the active ingredient is bound to an ion exchange resin which, optionally, can be coated with a diffusion barrier coating to modify the release properties of the resin.

The active compound may also be administered parenterally or intraperitoneally. Dispersions can also be prepared in glycerol, liquid, polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions (where water soluble) or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating such as lecithin, by the maintenance of the required particle size in the case of dispersions and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active compound in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of sterile powders for the preparation of sterile injectable solutions, the preferred methods of preparation are vacuum drying the freeze-drying technique plus any additional desired ingredient from previously sterile-filtered solution thereof.

As used herein, "pharmaceutically acceptable carrier" includes any and all solvents, dispersion media, coatings, antibacterial and antifungal agent, isotonic and absorption delaying agents for pharmaceutical active substances as well known in the art. Except insofar as any conventional media or agent is incompatible with the active ingredient, its use in the therapeutic compositions is contemplated. Supplementary active ingredients can also be incorporated into the compositions.

It is especially advantageous to formulate parenteral compositions in dosage unit form or ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specifics for the novel dosage unit forms of the invention are dictated by and directly dependent on (a) the unique characteristics of the active material an the particular therapeutic effect to be achieved, and (b) the limitations inherent in the art of compounding such as active material for the treatment of disease in living subjects having a diseased condition in which bodily health is impaired as herein disclosed in detail.

The principal active ingredient is compounded for convenient and effective administration in effective amounts with a suitable pharmaceutically acceptable carrier in dosage unit form as hereinbefore described. A unit dosage form can, for example, contain the principal active compound in amounts ranging from about 10 mg to about 6 g. Expressed in proportions, the active compound is generally present in from about 1 to about 750 mg/ml of carrier. In the case of compositions containing supplementary active ingredients, the dosages are determined by reference to the usual dose and manner of administration of the said ingredients.

As used herein the term "patient" or "subject" refers to a warm blooded animal, and preferably mammals, such as, for example, cats, dogs, horses, cows, pigs, mice, rats and primates, including humans. The preferred patient is a human.

The term "treat" refers to either relieving the pain associated with a disease or condition, to curing or alleviating the patient's disease or condition.

The compounds of the present invention are administered to a patient suffering from the aforementioned type of disorder in an effective amount. These amounts are equivalent to the therapeutically effective amounts described hereinabove.

The following example shows the properties of SPM 927 potentiating the prepulse inhibition of the antipsychotic drug clozapine in mice when administered together.

The used substance was SPM 927 which is the synonym for Harkoseride. The standard chemical nomenclature is (R)-2-acetamide-N-benzyl-3-methoxypropionamide. The international non-proprietory name of SPM 927 is lacosamide.

### Example

### The effects of SPM 927 alone and in combination with clozapine in an animal model for schizophrenia.

Some anti-epileptic drugs have proven pre-clinical and/or clinical efficacy as add-on therapy for schizophrenia. SPM 927 was tested in a simple animal model with predictive validity for schizophrenia (i.e. prepulse inhibition (PPI) of the acoustic startle reflex). SPM 927 was tested alone and in combination with the atypical antipsychotic drug clozapine.

The acoustic startle is an unconditioned reflex response to an external auditory stimulation. PPI refers to the reduction in the startle response caused by the presentation of a low-intensity auditory stimulus prior to the startle stimulus. The PPI paradigm is the choice for the study of schizophrenia and antipsychotic action due to the similarities between the results from human and rodent studies. Antipsychotic drugs, such as clozapine, result in a dose-dependent increase in PPI in mice. Thus, an increase of PPI in normal mice may be indicative of antipsychotic efficacy.

C57BL/6J mice obtained from the Jackson Laboratory, Bar Harbor, Maine were received at the age of 6 weeks and were assigned unique identification numbers (tail marked). Animals were housed 4 per cage in polycarbonate cages with filter tops and acclimated for 7 days. All animals were examined, handled, and weighed prior to initiation of the study to assure adequate health and suitability and to minimize non-specific stress associated with manipulation.

During the course of the study, 12/12 light/dark cycles and a room temperature of 20 to 23°C were maintained with a relative humidity maintained around 50%. Chow and water were provided ad libitum for the duration of the study. Each mouse was randomly assigned across the treatment groups and balanced by cage numbers. The test was performed during the animal's light cycle phase. Twelve animals were used in each treatment group.

SPM 927 was dissolved in Saline (0.9% NaCl in sterile H₂O). Clozapine was dissolved in 1 % Tween 80 in sterile H₂O at a final pH of 6.0. All compounds were administered intraperitoneal (ip) (volume injection: 10 ml/kg). SPM 927 was administered at doses of 1, 10 and 30 mg/kg and Clozapine at a dose of 3 mg/kg. Doses are expressed as mg of salt. Control mice were administered saline (vehicle 1, VEH 1) or 1% Tween 80 (vehicle 2, VEH 2) at a pH of 6.0. The drug treatments were balanced across days and the animals only used once. All tests were conducted blind. All compounds were administered 30 mins prior to testing.

Animals were placed in the PPI chambers (Med Associates) for a 5 min session of white noise (70 dB) habituation. After the acclimation period the test session was automatically started. The session started with an habituation block of 6 presentations of the startle stimulus alone, followed by 10 PPI blocks of 6 different types of trials. Trial types are: null (no stimuli), startle (120 dB), startle plus prepulse (4, 8 and 12 dB over background noise i.e. 74, 78 or 82 dB) and prepulse alone (82 dB). Trial types were presented at random within each block. Each trial started with a 50 ms null period during which baseline movements are recorded. There was a subsequent 20 ms period during which prepulse stimuli were presented and responses to the prepulse measured. After further 100 ms the startle stimuli were presented for 40 ms and responses recorded for 100 ms from startle onset. Responses were sampled every ms. The inter-trial interval was variable with an average of 15 s (range from 10 to 20 s). In startle alone trials the basic auditory startle is measured and in prepulse plus startle trials the amount of inhibition of the normal startle is determined and expressed as a percentage of the basic startle response (from startle alone trials), excluding the startle response of the first habituation block. Eight animals were tested at one time.

Twelve animals were tested in each group. A total of 108 mice were tested using following design:
VEH1/VEH2
SPM 927 [3 mg/kg] / VEH2
SPM 927 [10 mg/kg] / VEH2
SPM 927 [30 mg/kg] / VEH2
VEH1 / Clozapine [3 mg/kg]
SPM 927 [3 mg/kg] / Clozapine [3 mg/kg]
SPM 927 [10 mg/kg] / Clozapine [3 mg/kg]
SPM 927 [30 mg/kg] / Clozapine [3 mg/kg]

A two-way ANOVA (analysis of variance) with treatments as independent factor and pre-pulse intensities as dependent factor (i.e. repeated measure) was performed. This was followed by the post-hoc Newman Keuls test where indicated. A p < 0.05 was considered significant. The Newman Keuls test allows for the comparison of two independent samples.

Animals injected with clozapine (3 mg/kg) showed a significant improvement in prepulse inhibition in comparison to animals receiving vehicle (p<0.05, table 1). When administered alone, SPM 927 did not have any significant effect on prepulse inhibition at any dose tested, when compared to vehicle. The administration of SPM 927 (at 30 mg/kg but not at 3 or 10 mg/kg) in combination with clozapine however, did potentiate the effect of clozapine alone on prepulse inhibition i.e. the prepulse inhibition was significantly higher than the effect of clozapine alone (p<0.05).

The data obtained provide a basis for clinical trials in humans using SPM 927 as an add-on therapy for the treatment of schizophrenia.

**Table 1 Effects of SPM 927 alone and in combination with clozapine on prepulse inhibition of the acoustic startle response (PPI)**

| **Treatment** | **Mean PPI** |
|---|---|
| VEH 1/VEH 2 | 29±3 |
| VEH 1/CLOZ [3 mg/kg] | 45±2* |
| SPM 927 [3 mg/kg]/VEH 2 | 29±3 |
| SPM 927 [10 mg/kg]/VEH 2 | 34±3 |
| SPM 927 [30 mg/kg]NEH 2 | 27±2 |
| SPM 927 [3 mg/kg]/ CLOZ [3 mg/kg] | 43±2* |
| SPM 927 [10 mg/kg]/ CLOZ [3 mg/kg] | 45±2* |
| SPM 927 [30 mg/kg]/ CLOZ [3 mg/kg] | 57±5*,# |

| | |
|---|---|
| * p<0.05 for comparison to VEH/VEH group | |
| # p<0.05 for comparison to VEH/CLOZ group | |

## Claims

1. Use of at least one compound having the Formula (Ib) 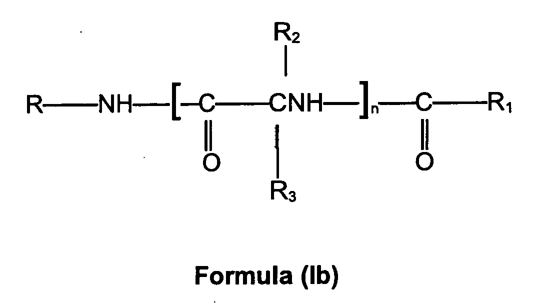 wherein
R is hydrogen, lower alkyl, lower alkenyl, lower alkynyl, aryl, aryl lower alkyl, heterocyclic, heterocyclic lower alkyl, lower alkyl heterocyclic, lower cycloalkyl or lower cycloalkyl lower alkyl, and R is unsubstituted or is substituted with at least one electron withdrawing group or/and at least one electron donating group;
R₁ is hydrogen or lower alkyl, lower alkenyl, lower alkynyl, aryl lower alkyl, aryl, heterocyclic lower alkyl, lower alkyl heterocyclic, heterocyclic, lower cycloalkyl, lower cycloalkyl lower alkyl, each unsubstituted or substituted with at least one electron donating group or/and at least one electron withdrawing group;
R₂ and R₃ are independently hydrogen, lower alkyl, lower alkenyl, lower alkynyl, aryl lower alkyl, aryl, halo, heterocyclic, heterocyclic lower alkyl, lower alkyl heterocyclic, lower cycloalkyl, lower cycloalkyl lower alkyl, or Z-Y wherein R₂ and R₃ may be unsubstituted or substituted with at least one electron withdrawing group or/and at least one electron donating group; and wherein heterocyclic in R₂ and R₃ is furyl, thienyl, pyrazolyl, pyrrolyl, methylpyrrolyl, imidazolyl, indolyl, thiazolyl, oxazolyl, isothiazolyl, isoxazolyl, piperidyl, pyrrolinyl, piperazinyl, quinolyl, triazolyl, tetrazolyl, isoquinolyl, benzofuryl, benzothienyl, morpholinyl, benzoxazolyl, tetrahydrofuryl, pyranyl, indazolyl, purinyl, indolinyl, pyrazolindinyl, imidazolinyl, imidazolindinyl, pyrrolidinyl, furazanyl, N-methylindolyl, methylfuryl, pyridazinyl, pyrimidinyl, pyrazinyl, pyridyl, epoxy, aziridino, oxetanyl, azetidinyl or, when N is present in the heterocyclic, an N-oxide thereof;
Z is O, S, S(O)ₐ, NR₄, NR₆' or PR₄ or a chemical bond;
Y is hydrogen, lower alkyl, aryl, aryl lower alkyl, lower alkenyl, lower alkynyl, halo, heterocyclic, heterocyclic lower alkyl, lower alkyl heterocyclic and Y may be unsubstituted or substituted with at least one electron donating group or/and at least one an electron withdrawing group, wherein heterocyclic has the same meaning as in R₂ or R₃ and, provided that when Y is halo, Z is a chemical bond, or ZY taken together is NR₄NR₅R₇, NR₄OR₅, ONR₄R₇, OPR₄R₅, PR₄OR₅, SNR₄R₇, NR₄SR₇, SPR₄R₅, PR₄SR₇, NR₄PR₅R₆, PR₄NR₅R₇, or N⁺R₅R₆R₇, 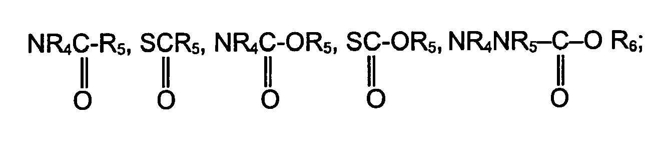 R₆' is hydrogen, lower alkyl, lower alkenyl, or lower alkynyl which may be unsubstituted or substituted with at least one electron withdrawing group or/and at least one electron donating group;
R₄, R₅ and R₆ are independently hydrogen, lower alkyl, aryl, aryl lower alkyl, lower alkenyl, or lower alkynyl, wherein R₄, R₅ and R₆ may independently be unsubstituted or substituted with at least one electron withdrawing group or/and at least one electron donating group; and
R₇ is R₆ or COOR₈ or COR₈, which R₇ may be unsubstituted or substituted with at least one electron withdrawing group or/and at least one electron donating group;
R₈ is hydrogen or lower alkyl, or aryl lower alkyl, and the aryl or alkyl group may be unsubstituted or substituted with at least one electron withdrawing group or/and at least one electron donating group; and
n is 1-4; and
a is 1-3,
or of a pharmaceutically acceptable salt thereof,
for the preparation of a pharmaceutical composition useful for the prevention, alleviation or/and treatment of schizophrenia in an add-on therapy.

2. Use according to claim 1 wherein one of R₂ and R₃ is hydrogen.

3. Use according to any one of claims 1-2 wherein n is 1.

4. Use according to any one of claims 1-3 wherein one of R₂ and R₃ is hydrogen and n is 1.

5. Use according to any one of claims 1-4 wherein R is aryl lower alkyl and R₁ is lower alkyl.

6. Use according to any one of claims 1-5 wherein
R₂ and R₃ are independently hydrogen, lower alkyl, or ZY;
Z is O, NR₄ or PR₄;
Y is hydrogen or lower alkyl or
ZY is NR₄NR₅R₇, NR₄OR₅, ONR₄R₇,

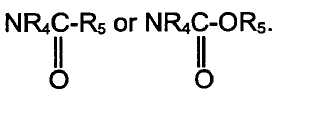

7. Use according to claim 6 wherein R₂ is hydrogen and and R₃ is lower alkyl, or ZY;
Z is O, NR₄ or PR₄;
Y is hydrogen or lower alkyl;
ZY is NR₄NR₅R₇, NR₄OR₅, ONR₄R₇,

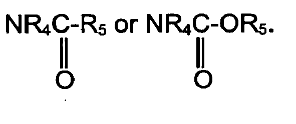

8. Use according any one of claims 1-7 wherein R₂ is hydrogen and R₃ is lower alkyl, which may be substituted or unsubstituted with at least one electron donating group or/and at least one electron withdrawing group, NR₄OR₅, or ONR₄R₇.

9. Use according to any one of claims 1-8 wherein R₃ is lower alkyl which is unsubstituted or substituted with hydroxy or loweralkoxy, NR₄OR₅ or ONR₄R₇, wherein R₄, R₅ and R₇ are independently hydrogen or lower alkyl, R is aryl lower alkyl, which aryl group may be unsubstituted or substituted with at least one electron withdrawing group and R₁ is lower alkyl.

10. Use according to any one of claims 1-9 wherein aryl is phenyl and is unsubstituted or substituted with halo.

11. Use according to any of claims 1-10 wherein the compound is
(R)-2-acetamido-N-benzyl-3-methoxy-propionamide;
O-methyl-N-acetyl-D-serine-m-fluorobenzylamide;
O-methyl-N-acetyl-D-serine-p-fluorobenzylamide;
N-acetyl-D-phenylglycinebenzylamide;
D-1,2-(N, O-dimethylhydroxylamino)-2-acetamide acetic acid benzylamide; or
D-1,2-(O-methylhydroxylamino)-2-acetamido acetic acid benzylamide.

12. Use of any one of claims 1-11 wherein the compound has the Formula (IIb) 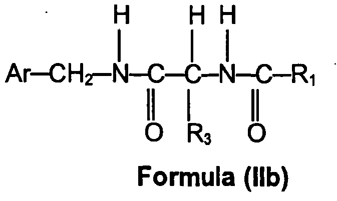 wherein
Ar is phenyl which is unsubstituted or substituted with at least one halo group;
R₃ is CH₂-Q, wherein Q is lower alkoxy containing 1-3 carbon atoms and R₁ is lower alkyl containing 1-3 carbon atoms
or of a pharmaceutically acceptable salt thereof.

13. Use according to claim 12 wherein Ar is unsubstituted phenyl.

14. Use according to claims 12 or 13 wherein halo is fluoro.

15. Use according to claims 12 to 14 wherein R₃ is CH₂-Q, wherein Q is alkoxy containing 1-3 carbon atoms and Ar is unsubstituted phenyl.

16. Use of any one of claims 1 to 15 wherein the compund is in the R configuration and has the formula 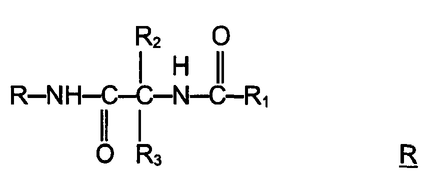 wherein
R is benzyl which is unsubstituted or substituted with at least one halo group;
R₃ is CH₂-Q, wherein Q is lower alkoxy containing 1-3 carbon atoms and R₁ is methyl
or a pharmaceutically acceptable salt thereof.

17. Use according to claim 16 which is substantially enantiopure.

18. Use according to claims 16 or 17 wherein R is unsubstituted benzyl.

19. Use according to claims 16 to 18 wherein halo is fluoro.

20. Use according to claims 16 to 19 wherein R₃ is CH₂-Q, wherein Q is alkoxy containing 1-3 carbon atoms and R is unsubstituted benzyl.

21. Use according to claim 1, wherein the compound of Formula (Ib) is (R)-2-Acetamido-N-benzyl-3-methoxypropionamide or a pharmaceutically acceptable salt thereof.

22. Use according to claim 21 wherein the compound is substantially enantiopure.

23. Use according to any one of the preceding claims, wherein the pharmaceutical composition is prepared for treatment with doses of the compound at least of 100 mg/day, preferably at least of 200 mg/day, more preferably at least of 300 mg/day, most preferably at least of 400 mg/day.

24. Use according to any one of the preceding claims, wherein the pharmaceutical composition is prepared for treatment with doses of the compound at a maximum of 6 g/day, more preferably at a maximum of 1 g/day and most preferably at a maximum of 600 mg/day.

25. Use according to any one of the preceding claims, wherein the pharmaceutical composition is prepared for treatment with increasing daily doses until a predetermined daily dose is reached which is maintained during the further treatment.

26. Use according to any one of the preceding claims, wherein the pharmaceutical composition is prepared for treatment in three doses per day, preferably two doses per day, more preferably in a single dose

27. Use according to any one of the preceding claims, wherein the pharmaceutical composition is prepared for an administration resulting in a plasma concentration of 0.1 to 15 µg/ml (trough) and 5 to 18.5 µg/ml (peak), calculated as an average over a plurality of treated subjects.

28. Use according to any one of the preceding claims, wherein the pharmaceutical composition is prepared for oral or i.v. administration.

29. Use according to any one of the preceding claims, wherein the pharmaceutical composition for prevention, alleviation or/and treatment of schizophrenia comprises
(a) at least one agent useful for prevention, alleviation or/and treatment of schizophrenia, and
(b) at least one compound as defined in any one of claims 1 to 22.

30. Use according to any one of the preceding claims, wherein the pharmaceutical composition comprises a single dose form or comprises a separate dose form comprising
(a) a first composition comprising at least one agent useful for prevention, alleviation or/and treatment of schizophrenia, and
(b) a second composition comprising at least one compound as defined in any of the claims 1 to 22.

31. Use according to claims 29 or 30, wherein the at least one agent useful for the prevention, alleviation or/and treatment of schizophrenia is at least one antipsychotic agent.

32. Use according to claim 31, wherein the at least one antipsychotic agent is an atypical antipsychotic agent, preferably clozapine, risperidone, aripiprazole, quietiapine or/and olanzapine.

33. A pharmaceutical composition comprising at least one compound as defined in any of the claims 1 to 22 useful for the prevention, alleviation or/and treatment of schizophrenia in an add-on therapy.

34. The pharmaceutical composition according to claim 33 comprising
(a) at least one agent useful for prevention, alleviation or/and treatment of schizophrenia, and
(b) at least one compound as defined in any of the claims 1 to 22.

35. The pharmaceutical composition according to claims 33 or 34 which comprises a single dose form or comprises a separate dose form comprising
(a) a first composition comprising at least one agent useful for prevention, alleviation or/and treatment of schizophrenia, and
(b) a second composition comprising at least one compound as defined in any of the claims 1 to 22.

36. The pharmaceutical composition according to claims 34 or 35, wherein the at least one agent useful for the prevention, alleviation or/and treatment of schizophrenia is at least one antipsychotic agent.

37. The pharmaceutical composition according to claim 36, wherein the at least one antipsychotic agent is an atypical antipsychotic agent, preferably clozapine, risperidone, aripiprazole, quietiapine or/and olanzapine.
